# EUROPEAN PATENT APPLICATION

(11) **EP 3 692 844 A1**
(43) Date of publication of application: **12.08.2020**
(21) Application number: 19155903.8
(22) Date of filing: 07.02.2019
(51) Int. Cl.: A24F 47/00, A61M 15/00

(54) **SMOKING SUBSTITUTE APPARATUS**

(71) Applicant: NERUDIA LIMITED, Liverpool L24 9HP (GB)
(72) Inventor: The designation of the inventor has not yet been filed
(74) Representative: Mewburn Ellis LLP

(57) **Abstract**

A smoking substitute apparatus, comprising: a first compartment containing an aerosol former; a second compartment in fluid communication with the first compartment, the second compartment is configured to receive one or more capsules each containing a property modifying agent; wherein the second compartment is configured to release said property modifying agent from the capsules in the second compartment into the aerosol former in the first compartment.

## Description

### Field of the Invention

The present invention relates to a smoking substitute apparatus and, in particular, a smoking substitute apparatus that is able to deliver flavour to a user by adding a flavourant to an aerosol former.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Combustion of organic material such as tobacco is known to produce tar and other potentially harmful byproducts. There have been proposed various smoking substitute systems in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. There are a number of different categories of smoking substitute systems, each utilising a different smoking substitute approach.

One approach is the so-called "vaping" approach, in which a vaporisable liquid, typically referred to (and referred to herein) as "e-liquid", is heated by a heating device (referred to herein as an electronic cigarette or "e-cigarette" device) to produce an aerosol vapour which is inhaled by a user. The e-liquid typically includes a base liquid as well as nicotine and/or a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu™ e-cigarette. The myblu™ e-cigarette is a closed system which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heater, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, e-liquid is heated by a heating device to produce an aerosol vapour which is inhaled by a user. Many e-cigarettes also deliver flavour to the user to enhance the experience. In such e-cigarettes, e-liquid is often sold as a flavoured product, e.g. a specific blend of flavour compounds are already homogeneously mixed with the e-liquid during the manufacturing process. As such, the user would have to purchase flavoured consumables available on the market with limited opportunities to personalise the vaping experience according to their preferences. Further, when blended in the e-liquid, flavour components may interact with other constituents in the e-liquid during storage. In addition, it may put additional strain on the supply chain for distributing a large variety of consumables each having different flavours.

There may be a need for improved design of smoking substitute systems, in particular in regards to the delivery of flavour to a user.

The present disclosure has been devised in the light of the above considerations.

### Summary of the Invention

At its most general, the present invention relates to a smoking substitute apparatus have separate storage for aerosol former and property modifying agent and means to release said property modifying agent to the aerosol former. This may allow the user to flavour the aerosol former at the point of use. Therefore the smoking substitute apparatus may enable the property modifying agent to be kept separate to the aerosol former, it may minimise the interaction between the two during transport and storage. Further, it may allow the user to specify the quantity and type of property modifying agent to be added to the aerosol former, thus it may enable the user to create an aerosol former with a specific flavour and/or colour tailored to the user's needs.

According to a first aspect there is provided a smoking substitute apparatus, comprising:
a first compartment containing an aerosol former;
a second compartment in fluid communication with the first compartment, the second
compartment is configured to receive one or more capsules each containing a property modifying agent;
wherein the second compartment is configured to release said property modifying agent from the capsules in the second compartment into the aerosol former in the first compartment.

The aerosol former may be an e-liquid. The e-liquid may, for example, comprise a base liquid and nicotine. The base liquid may include propylene glycol and/or vegetable glycerine. The e-liquid may be flavourless. That is, the e-liquid may not contain any flavourant and may consist solely of a base liquid of propylene glycol and/or vegetable glycerine and nicotine.

The property modifying agent may comprise a flavourant. The flavourant may be provided in solid, gel or liquid form. It may include menthol, liquorice, chocolate, fruit flavour (including e.g. citrus, cherry etc.), vanilla, spice (e.g. ginger, cinnamon) and tobacco flavour. The flavourant may modify a flavour of the aerosol former upon contacting or mixing with the aerosol former.

The property modifying agent may be a colorant. The colorant may be provided in solid, gel or liquid form. The colorant may modify a colour of the aerosol former upon contacting or mixing with the aerosol former. Advantageously, when the colorant is provided with the flavourant in the property modifying agent, the colorant may provide visual indication to a user for indicating a progress of release of property modifying agent. For example, the concentration of the flavourant may correspond to a change in colour of the aerosol former.

The capsules may allow the property modifying agent to be stored therein, and therefore be kept separately to the aerosol former stored in the first compartment during transportation and storage. The aerosol former and the property modifying agent may only come into contact once the property modifying agent is released from the capsules, e.g. by a releasing mechanism. This may advantageously eliminate or reduce the interaction between the aerosol former and the property modifying agent during transportation and storage. For example, the e-liquid may be flavoured with flavourant immediately before use.

The capsules may be configured to release the property modifying agent into the aerosol former using a releasing mechanism. More specifically, the capsule may have a capsule shell for containing or encapsulating the property modifying agent. By breaching or breaking the capsule shell, the property modifying agent as contained in the capsule may leak or flow out of the breached capsule shell and into the second compartment. Since the second compartment is in fluid communication with the first compartment, this allows the property modifying agent to flow into the first compartment to come into contact with the aerosol former stored in the first compartment. Alternatively or in addition, the aerosol former in the first compartment may flow into the second compartment to come into contact with the property modifying agent released in the second compartment. Advantageously, this allows a user to control the release of the property modifying agent at the point of use by activating the releasing mechanism.

The capsules may comprises capsule shells that are formed from any one of hydrolysed collagen, Hydroxy Propyl Methyl Cellulose, starch, Hydroxy Propyl Cellulose, PVA, Alginate or an extract from seaweed. More specifically, they are not soluble in the property modifying agent and therefore, they remain stable in storage. The capsule shells may be breached by rupturing the capsules. Alternatively or in addition, the capsule shells is soluble in the aerosol former such that the capsule shells may be breached by putting the capsules in contact with the aerosol former so as to dissolve the capsule shells.

The first compartment may be separated from the second compartment by a partition wall, wherein an opening is provided in the partition wall for effecting said fluid communication. Alternatively or in addition, the first compartment may be configured to be in fluid communication with the second compartment via a conduit. The opening and/or the conduit may be sized to allow passage of property modifying agent and the aerosol former, and it may be sized to prevent the capsules stored in the second compartment from entering the first compartment. Furthermore, the opening and/or the conduit may prevent the passage of breached capsule shells, i.e. the opening and/or conduit serves as a filter for the capsule shells. For example, the breached capsule shells may be retained in the second compartment. The first compartment may be adjacent to the second compartment or it may be spaced from the second compartment.

Optionally, the second compartment comprises one or more flexible walls, wherein moving the one or more flexible walls causes said one or more flexible walls to compress or ground the capsules stored in the second compartment. Said one or more flexible walls of the second compartment may be deformed when a force is applied thereon. For example, the flexible walls may form from elastic material that deforms elastically in reaction to a force. Or they may deform plastically such that the second compartment does not recover its original shape once the force is removed.

Optionally, said moving comprises biasing the one or more flexible walls towards a wall of the second compartment. More specifically, said moving may comprise biasing a plurality of flexible walls of the second compartment towards each other. For example, a user may squeeze or press on a pair of opposing flexible walls of the second compartment towards each other. Alternatively or in addition, it may comprise biasing one or more flexible walls towards a rigid wall of the second compartment. For example, a user may press on a designated portion of the second compartment to move said flexible wall towards a corresponding rigid wall. Either way, the user may bias the one or more flexible walls towards a wall of the second compartment to reduce the spacing in the second compartment, and by doing so the walls may exert compressive stress on the capsule and thereby ruptures the capsule. Advantageously, such arrangement allows the user to actively release the property modifying agent in a timely manner.

Optionally, said moving comprises twisting the smoking substitute apparatus about its longitudinal axis. For example, the second compartment may comprise two opposing flexible walls and by twisting the smoking substitute apparatus about its longitudinal axis, the two opposing flexible walls may urge towards each other. Doing so the flexible walls exerts a compressive stress and/or shear stress on the capsules, and thereby ruptures the capsules stored in the second compartment.

Optionally, the one or more flexible walls, of the second compartment comprises protrusions, e.g. ribs or embossed patterns such as dots, that aids the rupturing of the capsules. More specially, the internal surfaces, e.g. inner surfaces of walls and flexible walls, of the second compartment comprises protrusions or projections. For example, said protrusions may allow pressure to concentrate locally at their tips and therefore the capsules may be breached with better efficiency.

Optionally, the second compartment comprises one or more movable elements. For example, the moveable elements may comprise a piston provided in the second compartment, wherein moving the piston towards the capsules causes the piston to compress and thereby ruptures the capsule, thereby releasing the property modifying agent stored therein. For example, the one or more moveable elements may comprise a needle provided in the second compartment, wherein moving the needle towards the capsules causes the needle to pierce through the capsule shells of the one or more capsules, thereby releasing the property modifying agent stored therein. Moreover, the moveable element may be actuated by an actuator that extends outwardly from the smoking substitute apparatus, such that a user may rupture the capsule using the actuator. The actuator may be a lever, a toggle or a button that are mechanically or electrically linked to the moveable element. Therefore, movable element may be configured to move by pulling on the lever, or by toggling the toggle, or by applying pressure on the button.

Optionally, the second compartment comprises means for contacting the one or more capsules stored in the second compartment with the aerosol former, wherein upon contacting the aerosol former a soluble shell of each of the capsules is configured to dissolve in the aerosol former, and thereby releasing the property modifying agent stored therein. For example, a user may put smoking substitute apparatus in a given orientation, e.g. turning the apparatus upside down or tilting it, such that the aerosol former in the first compartment may flow, through an opening on a partition wall separating the first compartment and the second compartment, into the second compartment. The soluble shell of the microcapsule may dissolve instantaneously upon contacting the aerosol former so as to promptly release the property modifying agent, or it may gradually dissolve.

The opening, or the fluid communication between the first and second compartments, may be sealed by a removable seal having a sealing portion and a tab portion. As such, the releasing mechanism may be a removable seal that blocks said fluid communication during storage and transportation and may be configured to be removed by the user. More specifically, the sealing portion may be configured to cover and seal the opening and the tab portion is arranged to extend outwardly from smoking substitute apparatus. In use, a user may pull on the tab portion to disengage the sealing portion from the opening and thereby allows the aerosol former to flow into the second compartment for contacting the soluble shell of the capsules. This may cause the soluble shell to dissolve in the aerosol former and thereby releases the property modifying agent stored therein.

Optionally, the property modifying agent may comprise a solid, a gel, liquid or a gas. Or in other words, the property modifying agent may be in the form of a solid, a gel, a liquid or a gas. For example, the property modifying agent may be provided as a free dried solid powder which may readily dissolve in the aerosol former. The property modifying agent may be in the form of a gel that may readily dissolve in the aerosol former. The property modifying agent may be a liquid that may be miscible or immiscible with the aerosol former. The property modifying agent may be a gas that may be readily absorbed into the aerosol former once the two are put into contact. The property modifying agent may be a solid/liquid suspension.

Optionally, the second compartment may comprise a compartment inlet for receiving the one or more capsules. Advantageously, this may allow the capsules to be supplied separately to the smoking substitute apparatus. More advantageously, a user may select to provide one or more capsules each containing the same property modifying agent, or a mix of different property modifying agents, in a desired quantity, to be added to the aerosol former according to the user's preference.

Optionally, the compartment inlet at the second compartment may be provided with a manually operated valve or a flap, for example a hinged or sprung door which may be opened for receiving the property modifying agent and closed to seal the second compartment.

Optionally, the compartment inlet may be configured to engage with a dispenser outlet of a dispenser for dispensing the property modifying agent from the dispenser into the second compartment. Advantageously, this may allow the property modifying agent to be dispensed into the second compartment with greater efficiency and accuracy. The dispenser may comprise a prefilled tube, a canister or a tank or any other suitable dispenser. Optionally, the compartment inlet may only open when it is engaged with the dispenser outlet. For example, the flap or check valve provided at the compartment inlet may only open upon engaging with a corresponding mechanism at the dispenser outlet. The compartment inlet may reseal upon disengaging the dispenser outlet therefrom. Advantageously, this may ensure the second compartment is assessable only when a corresponding dispenser is presented, as such it may prevent accidental discharge of the capsules, as well as the tampering of the smoking substitute apparatus.

Optionally, the quantity of property modifying agent releasable to the aerosol former relates to the number of capsules that are received the second compartment. More specifically, the user may adjust the flavour and/or colour of the aerosol former by varying the number of capsules to be added to the second compartment, before releasing the property modifying agent from the capsules. For example, a larger quantity of capsules received in the second compartment would result in a larger quantity of property modifying agent to be released into the aerosol former. Advantageously, the flavour and/or colour of the aerosol former may be tailored to the user's preference.

Optionally, the smoking substitute apparatus comprises an outer wall, and wherein at least a portion of the outer wall is translucent or transparent such that the capsules and/or aerosol former stored in the respective first and/or second compartment are visible through said portion of the outer wall. Advantageously, this allows a user to inspect the condition of capsules stored in the second compartment, e.g. whether the capsules has been ruptured or dissolved, as well as visualising the progress of mixing the released property modifying agent and the aerosol former in the first compartment.

According to a second aspect there is provided a dispenser for an smoking substitute apparatus, comprising,
a storage for storing one or more capsules; the one or more capsules each contains a property modifying agent; and
a dispenser outlet in communication with the storage;
wherein the dispenser outlet is configured to engage with a compartment inlet of the smoking substitute apparatus so as to facilitate dispensing of the one or more capsules from the storage to a second compartment of the smoking substitute apparatus.

The dispenser may be provided as a prefilled tube, a canister or a tank. Therefore, when the dispenser outlet is connected to the compartment inlet, it may allow a specified number of capsules to flow in to the second compartment. Advantageously, this may allow the capsules containing property modifying agent to be dispensed into the second compartment with greater efficiency and accuracy.

Optionally, the dispenser may comprise a dosing mechanism for controlling the number of capsules that are dispensed through the dispenser outlet, and thereby controlling the quantity of property modifying agent that is releasable to an aerosol former that is stored in a first compartment of the smoking substitute apparatus.

Optionally, the dispenser may comprise an activator configured to activate the dispenser in proximity of the aerosol forming device. The activator may, for example, comprise a docking mechanism or a key which only allows microcapsules to flow when it is connected or engaged to, or in proximity of the smoking substitute apparatus. For example, the dispenser may be activated by pushing on and/or twisting a portion of the dispenser to open a door, a flap, or a check valve, e.g. diaphragm valve, a ball valve, a duckbill valve or other suitable valves. This may enable the capsules to flow in to the second compartment. When the dispenser is disengaged or removed from the aerosol forming device, the valves on both the dispenser outlet and the compartment inlet may be closed.

Optionally, the activator at the dispenser is configured to recognise and identify markings or signals corresponding to a smoking substitute apparatus, so as to confirm the presence of said apparatus in its proximity. Upon confirming the presence of said apparatus, the activator activates the dispenser, e.g. it actuates the valve at the dispenser outlet. Advantageously, such mechanism may prevent accidental discharge of property modifying agent, as well as prohibiting a user from tampering with the dispenser. As such that the dispenser only dispenses the property modifying agent in the presence of the aerosol forming device. For example, the activator may be an optical scanner configured to scan and recognise an image (e.g. a barcode or QR code) printed on a surface the aerosol forming device to confirm its presence. Alternatively, the activator may be an RF scanner configured to read and recognise RF signals originated from an RFID on at the aerosol forming device. Alternatively, the activator may respond to a magnetic field originated from a magnet at the aerosol forming device.

According to a third aspect of the present invention there is provided a smoking substitute kit, comprising:
the smoking substitute apparatus;
one or more capsules; and/or
the dispenser for dispensing the one or more capsules to the smoking substitute apparatus.

According to a fourth aspect of the present invention there is provided a method of adding property modifying agent to an aerosol former stored in a first compartment of a smoking substitute apparatus, comprising:
providing, in a second compartment of the smoking substitute apparatus in fluid communication with the first compartment, one or more capsules each containing the property modifying agent;
releasing the property modifying agent from the capsules in the second compartment to the aerosol former in the first compartment.

Optionally, said releasing comprises biasing one or more flexible walls of the second compartment towards a wall of the compartment.

Optionally, said releasing comprises twisting the smoking substitute device around its longitudinal axis.

Optionally, said providing may comprise dispensing the capsules to the second compartment.
Optionally, said providing may comprise dispensing the capsules to the second compartment using a dispenser.

Optionally, the method may comprise moving the aerosol forming device to facilitate mixing of the aerosol former and the property modifying agent once they are put into contact with each other.

The smoking substitute apparatus may be in the form of a consumable. The consumable may be configured for engagement with a main body (i.e. so as to form a closed smoking substitute system). For example, the consumable may comprise components of the system that are disposable, and the main body may comprise non-disposable or non-consumable components (e.g. power supply, controller, sensor, etc.) that facilitate the delivery of aerosol by the consumable. In such an embodiment, the aerosol former (e.g. e-liquid) may be replenished by replacing a used consumable with an unused consumable.

Alternatively, the smoking substitute apparatus may be a non-consumable apparatus (e.g. that is in the form of an open smoking substitute system). In such embodiments an aerosol former (e.g. e-liquid) of the system may be replenished by re-filling e.g. a reservoir of the smoking substitute apparatus with the aerosol former (rather than replacing a consumable component of the apparatus).

In light of this, it should be appreciated that some of the features described herein as being part of the smoking substitute apparatus may alternatively form part of a main body for engagement with the smoking substitute apparatus (i.e. when the smoking substitute apparatus is in the form of a consumable).

Where the smoking substitute apparatus is in the form of a consumable, the main body and the consumable may be configured to be physically coupled together. For example, the consumable may be at least partially received in a recess of the main body, such that there is an interference fit between the main body and the consumable. Alternatively, the main body and the consumable may be physically coupled together by screwing one onto the other, or through a bayonet fitting.

Thus, the smoking substitute apparatus may comprise one or more engagement portions for engaging with a main body. In this way, one end of the smoking substitute apparatus may be coupled with the main body, whilst an opposing end of the smoking substitute apparatus may define a mouthpiece of the smoking substitute system.

The smoking substitute apparatus may comprise a reservoir configured to store an aerosol former, such as an e-liquid. The e-liquid may, for example, comprise a base liquid and e.g. nicotine. The base liquid may include propylene glycol and/or vegetable glycerine. The e-liquid may be flavourless. That is, the e-liquid may not contain any flavourant and may consist solely of a base liquid of propylene glycol and/or vegetable glycerine and nicotine.

The reservoir may be in the form of a tank. At least a portion of the tank may be translucent. For example, the tank may comprise a window to allow a user to visually assess the quantity of e-liquid in the tank. A housing of the smoking substitute apparatus may comprise a corresponding aperture (or slot) or window that may be aligned with a translucent portion (e.g. window) of the tank. The reservoir may be referred to as a "clearomizer" if it includes a window, or a "cartomizer" if it does not.

The smoking substitute apparatus may comprise a passage for fluid flow therethrough. The passage may extend through (at least a portion of) the smoking substitute apparatus, between openings that may define an inlet and an outlet of the passage. The outlet may be at a mouthpiece of the smoking substitute apparatus. In this respect, a user may draw fluid (e.g. air) into and through the passage by inhaling at the outlet (i.e. using the mouthpiece). The passage may be at least partially defined by the tank. The tank may substantially (or fully) define the passage. In this respect, the tank may surround the passage.

The smoking substitute apparatus may comprise an aerosol-generator. The aerosol generator may comprise a wick. The aerosol generator may further comprise a heater. The wick may comprise a porous material. A portion of the wick may be exposed to fluid flow in the passage. The wick may also comprise one or more portions in contact with liquid stored in the reservoir. For example, opposing ends of the wick may protrude into the reservoir and a central portion (between the ends) may extend across the passage so as to be exposed to fluid flow in the passage. Thus, fluid may be drawn (e.g. by capillary action) along the wick, from the reservoir to the exposed portion of the wick.

The heater may comprise a heating element, which may be in the form of a filament wound about the wick (e.g. the filament may extend helically about the wick). The filament may be wound about the exposed portion of the wick. The heating element may be electrically connected (or connectable) to a power source. Thus, in operation, the power source may supply electricity to (i.e. apply a voltage across) the heating element so as to heat the heating element. This may cause liquid stored in the wick (i.e. drawn from the tank) to be heated so as to form a vapour and become entrained in fluid flowing through the passage. This vapour may subsequently cool to form an aerosol in the passage.

The smoking substitute apparatus (or main body engaged with the smoking substitute apparatus) may comprise a power source. The power source may be electrically connected (or connectable) to a heater of the smoking substitute apparatus (e.g. when engaged with the main body). The power source may be a battery (e.g. a rechargeable battery). A connector in the form of e.g. a USB port may be provided for recharging this battery.

When the smoking substitute apparatus is in the form of a consumable, the smoking substitute apparatus may comprise an electrical interface for interfacing with a corresponding electrical interface of the main body. One or both of the electrical interfaces may include one or more electrical contacts. Thus, when the main body is engaged with the consumable, the electrical interface may be configured to transfer electrical power from the power source to a heater of the consumable.

The electrical interface may also be used to identify the smoking substitute apparatus (in the form of a consumable) from a list of known types. For example, the consumable may have a certain concentration of nicotine and the electrical interface may be used to identify this. The electrical interface may additionally or alternatively be used to identify when a consumable is connected to the main body.

Again, where the smoking substitute apparatus is in the form of a consumable, the main body may comprise an interface, which may, for example, be in the form of an RFID reader, a barcode or QR code reader. This interface may be able to identify a characteristic (e.g. a type) of a consumable engaged with the main body. In this respect, the consumable may include any one or more of an RFID chip, a barcode or QR code, or memory within which is an identifier and which can be interrogated via the interface.

The smoking substitute apparatus or main body may comprise a controller, which may include a microprocessor. The controller may be configured to control the supply of power from the power source to the heater of the smoking substitute apparatus (e.g. via the electrical contacts). A memory may be provided and may be operatively connected to the controller. The memory may include non-volatile memory. The memory may include instructions which, when implemented, cause the controller to perform certain tasks or steps of a method.

The main body or smoking substitute apparatus may comprise a wireless interface, which may be configured to communicate wirelessly with another device, for example a mobile device, e.g. via Bluetooth®. To this end, the wireless interface could include a Bluetooth® antenna. Other wireless communication interfaces, e.g. WiFi®, are also possible. The wireless interface may also be configured to communicate wirelessly with a remote server.

A puff sensor may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the heater of the consumable in response to a puff detection by the sensor. The control may be in the form of activation of the heater in response to a detected puff. That is, the smoking substitute apparatus may be configured to be activated when a puff is detected by the puff sensor. When the smoking substitute apparatus is in the form of a consumable, the puff sensor may form part of the consumable or the main body.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
Figure 1A is a front view of a smoking substitute system, according to a first embodiment, in an engaged position;
Figure 1B is a front view of smoking substitute system of the first embodiment in a disengaged position;
Figure 1C is a section view of a smoking substitute apparatus of the first embodiment;
Figure 2A is a section view of a capsule of a second embodiment;
Figure 2B is a front view of the capsule being received in a smoking substitute apparatus of the second embodiment;
Figure 2C illustrates a method of releasing property modifying agent in the smoking substitute apparatus;
Figure 2D illustrates another method of releasing property modifying agent in the smoking substitute apparatus;
Figure 3 illustrates a capsule being inserted into the smoking substitute apparatus of another embodiment.
Figures 4A to 4C illustrates the use of a dispenser for dispensing a capsule to a smoking substitute apparatus.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

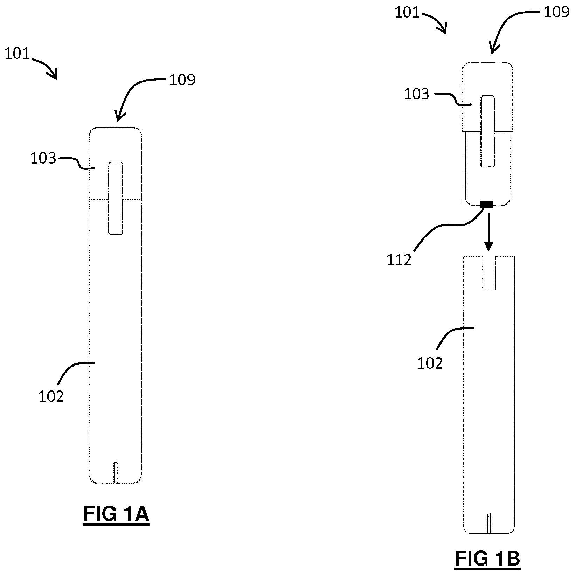

Figures 1A and 1B illustrate a smoking substitute system in the form of an e-cigarette system 101. The system 101 comprises an e-cigarette device defining a main body 102 of the system 101, and an smoking substitute apparatus in the form of an e-cigarette consumable (or "pod") 103. The smoking substitute apparatus is a smoking substitute apparatus. In the illustrated embodiment the consumable 103 (smoking substitute apparatus) is removable from the main body (e-cigarette device), so as to be a replaceable component of the system 101. In other words, the e-cigarette system 101 is a closed system.

As is apparent from Figures 1A and 1B, the consumable 103 is configured to engage the main body 102. Figure 1A shows the main body 102 and the consumable 103 in an engaged state, whilst Figure 1B shows the main body 102 and the consumable 103 in a disengaged state. When engaged, a portion of the consumable 103 is received in a cavity of the main body 102 and is retained in the engaged position by way of a snap-engagement mechanism. In other embodiments, the main body 102 and consumable 103 may be engaged by screwing one into (or onto) the other, through a bayonet fitting, or by way of an interference fit.

The system 101 is configured to vaporise an aerosol-former, which in the illustrated embodiment, is in the form of a nicotine-based e-liquid 104. The e-liquid 104 comprises nicotine and a base liquid including propylene glycol and/or vegetable glycerine. In the present embodiment, the e-liquid 104 is flavourless (and does not include any added flavourant). That is, if the e-liquid 104 were to be inhaled (i.e. in aerosol form) by a user, it would not have a particularly perceptible flavour or taste.

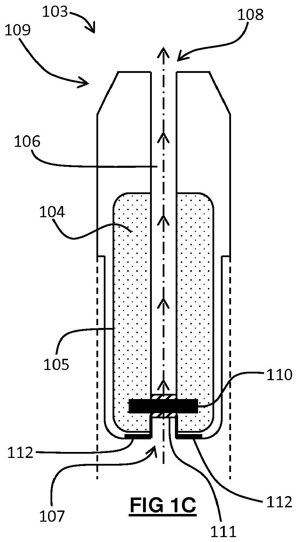

As is more apparent from Figure 1C, this e-liquid 104 is stored within a reservoir in the form of a tank 105 that forms part of the consumable 103. In the illustrated embodiment, the consumable 103 is a "single-use" consumable 103. That is, upon exhausting the e-liquid 104 in the tank 105, the intention is that the user disposes of the entire consumable 103. In other embodiments, the e-liquid (i.e. aerosol former) may be the only part of the system that is truly "single-use". That is, the tank may be refillable with e-liquid or the e-liquid may be stored in a non-consumable component of the system. For example, the e-liquid may be stored in a tank located in the main body or stored in another component that is itself not single-use (e.g. a refillable cartomizer).

The tank 105 surrounds, and thus defines a portion of, a passage 106 that extends between an inlet 107 and an outlet 108 at opposing ends of the consumable 103. In this respect, the passage comprises an upstream end at the end of the consumable 103 that engages with the main body 102, and a downstream end at an opposing end of the consumable 103 that comprises a mouthpiece 109 of the system 101. When the consumable 103 is engaged with the main body 102, a user can inhale (i.e. take a puff) via the mouthpiece 109 so as to draw air through the passage 106, and so as to form an airflow (indicated by arrows) in a direction from the inlet 107 to the outlet 108 of the passage 106. Although not illustrated, the passage 106 may be partially defined by a tube (e.g. a metal tube) extending through the consumable 103. The passage 106 is in fluid communication with a gap defined between the consumable 103 and the main body 102 (when engaged) such that air outside of the system 101 is drawn into the passage 106 (during an inhale).

The smoking substitute system 101 is configured to vaporise the e-liquid 104 for inhalation by a user. To provide this, the consumable 103 comprises a heater having of a porous wick 110 and a resistive heating element in the form of a heating filament 111 that is helically wound around a portion of the porous wick 110. The porous wick 110 extends across the passage 106 (i.e. transverse to a longitudinal axis of the passage106) and opposing ends of the wick 110 extend into the tank 105 (so as to be submerged in the e-liquid 104). In this way, e-liquid 104 contained in the tank 105 is conveyed from the opposing ends of the porous wick 110 to a central portion of the porous wick 110 so as to be exposed to the airflow in the passage 106 (i.e. caused by a user inhaling).

The helical filament 111 is wound about this exposed central portion of the porous wick 110 and is electrically connected to an electrical interface in the form of electrical contacts 112 mounted at the end of the consumable that is proximate the main body 102 (when engaged). When the consumable 103 is engaged with the main body 102, the electrical contacts 112 contact corresponding electrical contacts (not shown) of the main body 102. The main body electrical contacts are electrically connected to a power source (not shown) of the main body 102, such that (in the engaged position) the filament 111 is electrically connected to the power source. In this way, power can be supplied by the main body 102 to the filament 111 in order to heat the filament 111. This heat is transferred from the filament 111 to the porous wick 110 which causes e-liquid 104 conveyed by the porous wick 110 to increase in temperature to a point at which it vaporises. The vaporised e-liquid becomes entrained in the airflow and, between the vaporisation point at the filament 111 and the outlet 108 of the passage 106, condenses to form an aerosol. This aerosol is then inhaled, via the mouthpiece 109, by a user of the system 101.

The power source of the main body 102 may be in the form of a battery (e.g. a rechargeable battery). The main body 102 may comprise a connector in the form of e.g. a USB port for recharging this battery. The main body 102 may also comprise a controller that controls the supply of power from the power source to the main body electrical contacts (and thus to the filament 111). That, is the controller may be configured to control a voltage applied across the main body electrical contacts, and thus the voltage applied across the filament 111. In this way, the filament 111 may only be heated under certain conditions (e.g. during a puff and/or only when the system is in an active state). In this respect, the main body 102 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 102 and consumable 103 may comprise a further interface which may, for example, be in the form of an RFID reader, a barcode or QR code reader. This interface may be able to identify a characteristic (e.g. a type) of a consumable 103 engaged with the main body 102. In this respect, the consumable 103 may include any one or more of an RFID chip, a barcode or QR code, or memory within which is an identifier and which can be interrogated via the interface.

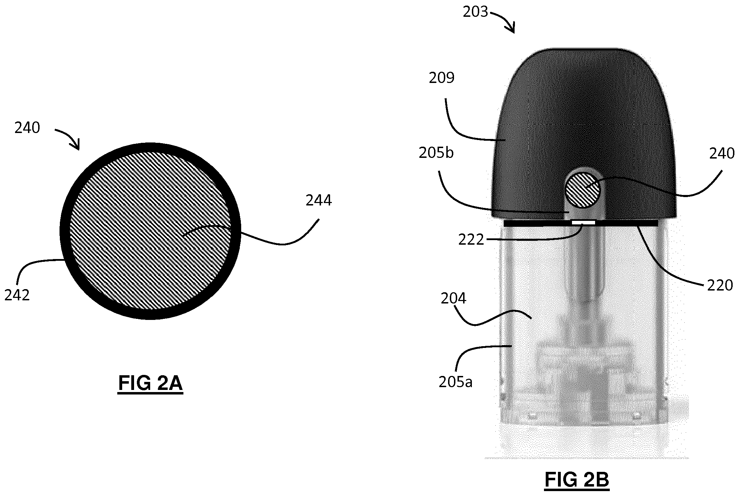

Figures 2A and 2B respectively shows a capsule 240 and a smoking substitute apparatus, or a consumable 203 for receiving the capsule 240 according to an embodiment of the present invention. The consumable 203 comprises a tank that is divided into a first compartment 205a and a second compartment 205b by a partition wall 220. In other words, the first compartment 205a and the second compartment 205b shares the partition wall 220. The partition wall 220 extends across the tank, e.g. from a sidewall of the tank towards an opposing sidewall of the tank, as such a base portion of the second compartment 205b is adjacent to an upper portion of the first compartment 205a.

The capsule 240 comprises a shell 242 encapsulating a body of property modifying agent 244. In the illustrated embodiment the shell 242 is formed from a hydrolysed collagen, such as a gelatine. The shell is insoluble in the property modifying agent, as such it remains stable during storage and transportation. The property modifying agent 244 in the illustrated embodiment is a liquid property modifying agent, and comprises a flavourant and a colourant respectively configured to modify the flavour and colour of the aerosol former 204 as stored in the first compartment 205a. In other embodiments, the property modifying agent may be a solid, a gel, a liquid or a gas, and may comprise only a flavourant without the presence of a colorant or may comprise a colorant without the presence of a flavourant. For example, the property modifying agent 244 may alternatively be a powder encapsulated in the shell 242.

In the illustrated embodiment, the first compartment 205a contains a supply of aerosol former, or e-liquid 204, and the second compartment 205b receives the capsule 240. That is, in this example, the consumable is provided with a capsule 240 contained in the second compartment 205b. The aerosol former 204 is in a liquid form. The aerosol former 204 comprises nicotine and a base liquid, e.g. propylene glycol. The aerosol former 204 does not contain any flavourant and therefore the aerosol former is not flavoured. The aerosol former 204 in this embodiment does not contain any colourant. That is, the aerosol former 204 is in the natural colour of the propylene glycol and the nicotine, and therefore it appears as a translucent or transparent liquid.

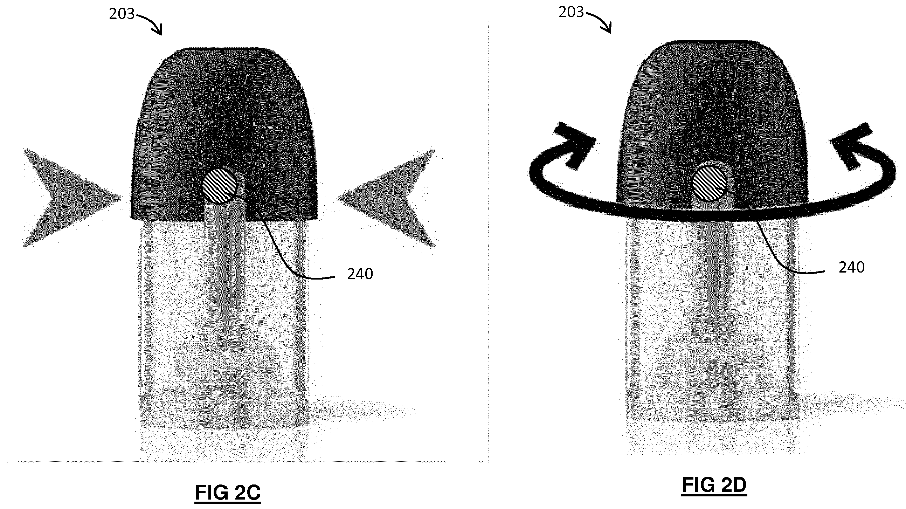

The partition wall 220 as shown in Figure 2 comprises an opening 222. The opening 222 effects fluid communication between the first compartment 205a and the second compartment 205b. However, the opening 222 is sized such that the capsule 240 as received in the first compartment 205a is retained therein, e.g. the opening 222 is sized smaller than the cross sectional diameter of the capsule 240. The opening 222 is unobstructed in the illustrated embodiment as shown in Figure 2. That is, the consumable 203 in this example is supplied with the aerosol former 204 freely flowable between the first compartment 205a and second compartment 205b, e.g. the aerosol former 204 may be in contact with the capsule 240 and is separated from the property modifying agent 244 encapsulated in the capsule shell 242.

The second compartment 205b of the consumable 203 in the illustrated embodiment comprises flexible sidewalls that deforms upon applying a force thereat. In some other embodiments, the consumable is formed from an elastic material and therefore both the first compartment and the second compartment comprises flexible sidewalls. Additionally, the mouthpiece 209 that surrounds the second compartment 205b is also formed from an elastic material such that the second compartment 205b may be configured to deform by compressing or twisting the mouthpiece 209.

To release the property modifying agent 244 from the capsule 240, a user may compress, e.g. squeeze on, the mouthpiece 209 so as to bias the flexible sidewalls against each other, as shown in Figure 2C. Doing so urges the flexible sidewalls towards the capsule 240 and thereby compresses the capsule 240. The compressive stress acting on the capsule 240 causes the capsule shell 242 to rupture, and thereby releases the body of property modifying agent 244 to the cavity of the second compartment 205b. The property modifying agent 244 then flows or drips, under gravity, through the opening 222 and into the aerosol former 204 stored in the first compartment 205a. In the illustrated embodiment, the ruptured capsule shell 242 may be retained in the second compartment 205b as its size precludes it form passing through the opening 222.

Alternatively, a user may twist the consumable 203 about its longitudinal axis, as shown in Figure 2D. Doing so reduces the spacing between the flexible sidewalls and thereby urges the flexible sidewalls towards the capsule 240 to compress it. The compressive stress acting on the capsule 240 causes the capsule shell 242 to rupture, and thereby releases the body of property modifying agent 244 to the cavity of the second compartment 205b. The property modifying agent 244 then flows or drips, under gravity, through the opening 222 and into the aerosol former 204 stored in the first compartment 205a. In the illustrated embodiment, the ruptured capsule shell 242 may be retained in the second compartment 205b as its size precludes it form passing through the opening 222. That is, the flexible sidewalls are configured to compress the capsule 240 stored in the second compartment 205b either by compressing the flexible sidewalls or by twisting the consumable 203.

In some other embodiments, the flexible sidewalls of the second compartment may comprise protrusion, in the form of ribs or projections for rupturing the capsules stored in the second compartment. The protrusions may allow pressure to build up at their tips, thereby allowing the capsules to be ruptured more efficiently.

In some other embodiments, the second compartment may comprise a moveable element for rupturing the capsule. The movable element may comprise an actuator that extends outwardly from the consumable that allows a user to move the moveable element and thereby to rupture the capsule. The movable element may be a movable wall positioned at an upper end of the second compartment away from the first compartment, and may configure to travel along the longitudinal axis of the consumable. In use, said movable wall may move towards the partition wall and thereby ruptures the capsules stored in the second compartment. That is, the moveable wall may function as a piston for rupturing the capsules. Alternatively or in addition, the movable element may comprise one or more needles in the second compartment that is configured to travel along the longitudinal axis of the consumable. In use, said needles may move towards the capsules to pierce through the capsule shell, and thereby releasing the property modifying agent to the second compartment.

In some other embodiments, the capsules may comprise a capsule shell that is soluble in the aerosol former. In such cases, the opening may be sealed by a removable seal in order to prevent the aerosol former from coming into contact with the capsule shell, and thereby prevents the capsule shell from dissolving in the aerosol former prior to seal removal. More specifically, a seal portion of the removable seal may cover the opening in a sealing position and thereby hermetically seals the opening. Therefore, in the sealing position, the seal portion of the removable seal prevents fluid communication between the first compartment and the second compartment. The removal seal is configured to be removed from the sealing position and thereby detaches the seal portion form the opening, and thereby establishes fluid communication between the first compartment and second compartment.

The removable seal may additionally comprise a tab portion in connection with the seal portion. That is, the seal portion may be formed together with the tab portion to form the removable seal. Said tab portion may extends, along a slot in the consumable and outwardly from the base of the consumable through a check valve, such that at least a part of the tab portion is accessible to a user. The tab portion comprises an enlarged portion external to the consumable for a user to grip onto.

Prior to use or in use, the user may pull on the tab portion away from the consumable to detach the seal portion from the opening. The removable seal may be completely removed from the consumable to be disposed of. Doing so establishes fluid communication between the first compartment and second compartment, thereby allowing the aerosol former in the first compartment to come into contact with the capsule in the second compartment. More specifically, the capsule shell of the capsules may dissolve in the aerosol former once they come into contact with each other. As such, the body of the property modifying agent may then be released into the aerosol former.

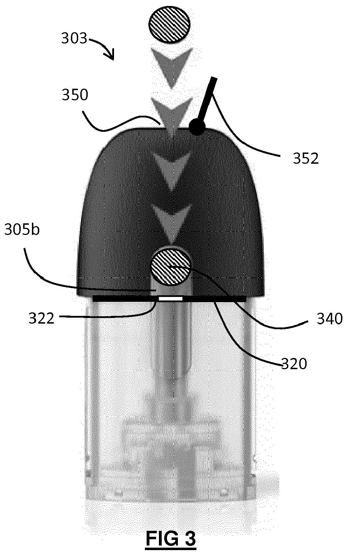

Figure 3 illustrates a consumable 303 according to another embodiment of the present invention. The consumable 303 is similar, functionally and structurally to the consumable 203 as shown in Figure 2B. The consumable 303 differs to the consumable 203 in that the consumable 303 comprises a compartment inlet 350 at the second compartment 305b for providing access to the second compartment 305b. More specifically, the compartment inlet 350 is sized to allow capsules 340 to be inserted through there and into the second compartment 305b. The compartment inlet 350 is closed by a flap 352 that is hingedly attached to the consumable 303 at a location adjacent to the compartment inlet 350. Therefore the flap 352 is pivotable between an opened position for providing access to the second compartment 350b and a closed position where the second compartment 350b is sealed off. When the flap 352 in the opened position, a user may add capsules 340, either manually or with the use of a dispenser, to the second compartment 205b.

The quantity of property modifying agent releasable to the aerosol former relates to the number of capsules that is being added to the second compartment 350b. For example, a user may add a predetermined number of capsules to the second compartment 350b in order to achieve the desired flavour and/or colour. The user may add a mix of capsules each having a different flavourant (e.g. blueberry flavourant in a first capsule and cherry flavourant in a second capsule) so as to achieve a mixed flavour in the aerosol former.

In some embodiment, the volume of the second compartment 350b is limited so as to limit the maximum amount of property modifying agent a user can add to the aerosol former. This prevent the user form overdosing the property modifying agent in the aerosol former.

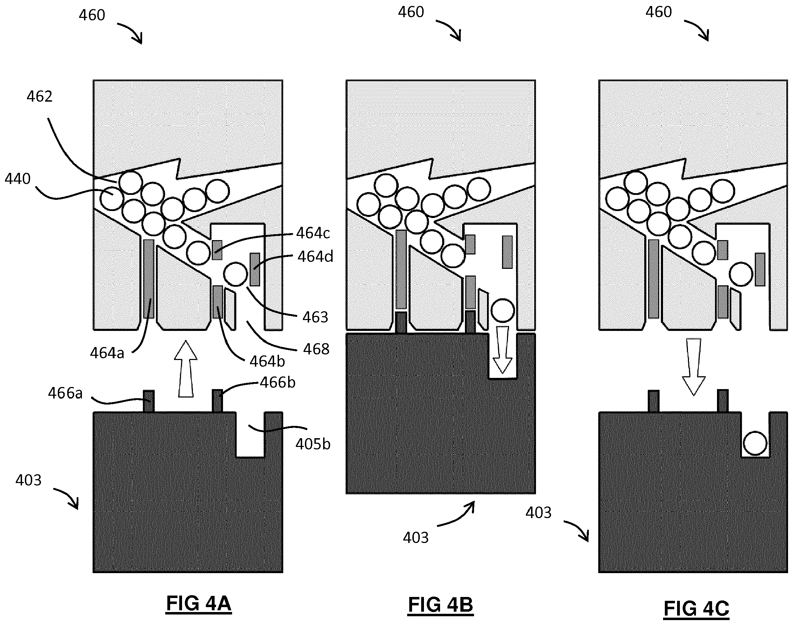

Figures 4a, 4b and 4c sequentially show a dispenser 460 dispensing a predetermined number of capsules 440 to a second compartment 405b of a consumable 403. Figure 4a and Figure 4c show the dispenser 460 being separated from the consumable 403 and Figure 4b shows the dispenser 460 is engaged with the consumable 403. The dispenser 460 comprises a storage 462 for storing a supply of capsules 440 and a dispensing chamber 463 for storing a predetermined number of capsules to be dispensed. For example, the dispensing chamber 463 is sized to receive the required number of capsules to be dispensed, in this case a single capsule. However the dispensing chamber may be sized to receive a plurality of capsules such that said plurality of capsules may be dispensed at once. When the dispenser is separated from the consumable, as shown in Figure 4a, a dispensing element 464d prevents the capsule from being dispensed from the dispensing chamber 463.

The dispenser comprises a dispensing mechanism, which is formed of dispensing elements 464a, 464b, 464c and 464d, and is configured to be activated by a corresponding activating mechanism 466a and 466b at the consumable 403. More specifically, the activating mechanism 466a and 466b are protrusions that, when the dispenser 460 is engaged with the consumable 403 as shown in Figure 4B, push onto the dispensing element 464a and 464b and thereby urges the dispensing mechanism towards an upward direction. This causes the dispensing element 464d to move and thereby allows a capsule to dispense through a dispenser outlet 468 into the second compartment 405b. Furthermore, when the dispensing mechanism is activated as shown in Figure 4B, a slot formed between the dispensing element 464b and 464c also moves in line with a capsule 440 stored in the storage 462 and thereby allowing the capsule in the storage 462 to drop into the dispensing chamber 463. That is, activating the dispensing mechanism causes a capsule to be dispensed from the dispensing chamber 463, as well as allowing a capsule form the storage 462 to replenish the dispensing chamber.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A smoking substitute apparatus, comprising:
a first compartment containing an aerosol former;
a second compartment in fluid communication with the first compartment, the second compartment is configured to receive one or more capsules each containing a property modifying agent;
wherein the second compartment is configured to release said property modifying agent from the capsules in the second compartment into the aerosol former in the first compartment.

2. The smoking substitute apparatus of claim 1, wherein the second compartment comprises one or more flexible walls, wherein moving the one or more flexible walls causes said one or more flexible walls to compress or ground the capsules stored in the second compartment.

3. The smoking substitute apparatus of claim 2, wherein said moving comprises biasing the one or more flexible walls towards a wall of the second compartment.

4. The smoking substitute apparatus of claim 2, wherein said moving comprises twisting the smoking substitute apparatus about its longitudinal axis.

5. The smoking substitute apparatus of any one of claims 2 to 4, wherein the one or more flexible wall of the second compartment comprises protrusions.

6. The smoking substitute apparatus of any one of the preceding claims, wherein second compartment comprises one or more movable elements.

7. The smoking substitute apparatus of any one of the preceding claims, wherein second compartment comprises means for contacting the one or more capsules stored in the second compartment with the aerosol former, wherein upon contacting the aerosol former a soluble shell of each of the capsules is configured to dissolve in the aerosol former, and thereby releasing the property modifying agent stored therein.

8. The smoking substitute apparatus of any one of the preceding claims, wherein the property modifying agent comprises any one or more of a solid, a gel, liquid or a gas.

9. The smoking substitute apparatus of any one of the preceding claims, wherein the property modifying agent comprises a flavourant and/or a colourant.

10. The smoking substitute apparatus of any one of the preceding claims, wherein the second compartment comprises a compartment inlet for receiving the one or more capsules.

11. The smoking substitute apparatus of claim 10, wherein the compartment inlet is configured to engage with a dispenser outlet of a dispenser for dispensing the property modifying agent from the dispenser into the second compartment.

12. The smoking substitute apparatus of any one of the preceding claims, wherein the quantity of property modifying agent releasable to the aerosol former relates to the number of capsules that are received the second compartment.

13. A dispenser for an smoking substitute apparatus, comprising,
a storage for storing one or more capsules; the one or more capsules each contains a property modifying agent; and
a dispenser outlet in communication with the storage;
wherein the dispenser outlet is configured to engage with a compartment inlet of the smoking substitute apparatus so as to facilitate dispensing of the one or more capsules from the storage to a second compartment of the smoking substitute apparatus.

14. A smoking substitute kit, comprising:
the smoking substitute apparatus of any one of the claims 1 to 12;
one or more capsules of any one of claims 1 to 12; and/or
the dispenser of claim 13 for dispensing the one or more capsules to the smoking substitute apparatus.

15. A method of adding property modifying agent to an aerosol former stored in a first compartment of a smoking substitute apparatus, comprising:
providing, in a second compartment of the smoking substitute apparatus in fluid communication with the first compartment, one or more capsules each containing the property modifying agent;
releasing the property modifying agent from the one or more capsules in the second compartment to the aerosol former in the first compartment.
